# EUROPEAN PATENT APPLICATION

(11) **EP 4 299 009 A1**
(43) Date of publication of application: **03.01.2024**
(21) Application number: 22759762.2
(22) Date of filing: 24.02.2022
(51) Int. Cl.: A61B 7/04

(54) **ELECTRONIC STETHOSCOPE**

(30) Priority: 26.02.2021 JP 2021030783
(71) Applicant: FUJIFILM Corporation, Tokyo 106-8620 (JP)
(72) Inventor: SASAKI, Tsutomu, Ashigarakami-gun, Kanagawa 258-8538 (JP)
(74) Representative: Meissner Bolte Partnerschaft mbB
(86) International application number: PCT/JP2022/007751
(87) International publication number: WO 2022/181727

(57) **Abstract**

An electronic stethoscope includes: a biological sound sensor that detects a biological sound and outputs an analog-format biological sound signal; an analog system that processes the biological sound signal without converting the biological sound signal into a digital signal and outputs the biological sound signal to an outside; and a digital system that converts the biological sound signal into the digital signal and outputs the biological sound signal.

## Description

### BACKGROUND OF THE INVENTION

### 1. Field of the Invention

The disclosed technology relates to an electronic stethoscope.

### 2. Description of the Related Art

The following technologies are known as technologies related to electronic stethoscopes. For example, JP1998-504748A (JP-H10-504748A) discloses digital stethoscope including a vibration transducer such as a microphone, a preamplifier that performs impedance conversion, an amplifier that performs preemphasis, an analog-to-digital converter, a unit composed of a digital filter and a digital-to-analog converter, an output amplifier, and a speaker.

### SUMMARY OF THE INVENTION

In conventional electronic stethoscopes, an analog-format biological sound signal output from a biological sound sensor comprising a piezoelectric material, a capacitor microphone, or the like that detects biological sounds, such as heart sounds and respiratory sounds, is converted into a digital signal, and then various types of signal processing, such as filtering processing and equalizing processing, is performed. The digital-format biological sound signal that has been subjected to the signal processing is converted into an analog signal and output as audio through an acoustic device, such as a headphone or an earphone.

In the conventional electronic stethoscopes, digital signal processing for compensating for the sensitivity characteristic of the biological sound sensor is essential. That is, piezoelectric ceramics or the like constituting the conventional biological sound sensor do not have sufficient sensitivity of 1 KHz or less, which is the main frequency range of the biological sound. In addition, capacitor microphones, piezoelectric ceramics, or piezoelectric materials generally have low dielectric losses, which make it difficult for sound to be converted into heat, and a reverberant sound is generated. Further, ambient noise also has a significant effect. Therefore, in the conventional electronic stethoscopes, digital signal processing for compensating for the above-described sensitivity characteristic of the biological sound sensor and improving sound quality is essential.

However, the biological sound signal is degraded by sampling and quantization during converting the biological sound signal into a digital signal. The biological sound perceived by reproducing the biological sound signal degraded by the digital conversion may deviate significantly from an original biological sound or an auscultation sound of conventional analog stethoscopes, which may cause adverse effects on diagnosis through auscultation.

The disclosed technology has been made in view of the above-described point, and an object of the disclosed technology is to provide an electronic stethoscope capable of improving sound quality of detected biological sounds and auscultation sounds.

According to the disclosed technology, there is provided an electronic stethoscope comprising: a biological sound sensor that detects a biological sound and outputs an analog-format biological sound signal; an analog system that processes the biological sound signal without converting the biological sound signal into a digital signal and outputs the biological sound signal to an outside; and a digital system that converts the biological sound signal into the digital signal and outputs the biological sound signal.

The biological sound sensor may contain a polymer-based piezoelectric composite material obtained by dispersing piezoelectric particles in a viscoelastic matrix consisting of a polymer material having viscoelasticity at room temperature.

In the electronic stethoscope, a first preamplifier that amplifies the biological sound signal and a second preamplifier that attenuates a high-frequency component included in the biological sound signal may further be provided, and an output signal of the second preamplifier may be distributed to the analog system and the digital system. It is preferable that the first preamplifier has an input impedance Z of 50 kS2 ≤ Z ≤ 10 MS2, and that the second preamplifier has a cutoff frequency fc of 1 kHz ≤ f_{C} ≤ 3 kHz and an attenuation slope A of 12 dB/oct ≤ A ≤ 36 dB/oct.

The second preamplifier may include an amplification unit and a filter unit. The amplification unit may include a first operational amplifier having a first inverting input terminal, a first non-inverting input terminal, and a first output terminal, a first resistance element of which one end is connected to an output end of the first preamplifier and the other end is connected to the first inverting input terminal, and a second resistance element of which one end is connected to the first inverting input terminal and the other end is connected to the first output terminal. The filter unit may include a second operational amplifier having a second inverting input terminal, a second non-inverting input terminal, and a second output terminal, a third resistance element of which one end is connected to the second non-inverting input terminal, a first capacitor of which one end is connected to the non-inverting input terminal and the other end is connected to a ground potential, a fourth resistance element of which one end is connected to the other end of the third resistance element, and a second capacitor of which one end is connected to a connection portion between the third resistance element and the fourth resistance element and the other end is connected to the second inverting input terminal and the second output terminal. It is preferable that a ratio R2/R1 of a resistance value R2 of the second resistance element to a resistance value R1 of the first resistance element is 1 ≤ R2/R1 ≤ 10, and that a ratio C2/C1 of an electrostatic capacitance C2 of the second capacitor to an electrostatic capacitance C1 of the first capacitor is 3 ≤ C2/C1 ≤ 15.

The second preamplifier may include a third operational amplifier having a third inverting input terminal, a third non-inverting input terminal, and a third output terminal, a fifth resistance element of which one end is connected to the third inverting input terminal, a sixth resistance element of which one end is connected to the other end of the fifth resistance element, a seventh resistance element of which one end is connected to the third output terminal and the other end is connected to a connection portion between the fifth resistance element and the sixth resistance element, a third capacitor of which one end is connected to the third inverting input terminal and the other end is connected to the third output terminal, and a fourth capacitor of which one end is connected to the connection portion between the fifth resistance element and the sixth resistance element and the other end is connected to a ground potential. It is preferable that a ratio R7/R6 of a resistance value R7 of the seventh resistance element to a resistance value R6 of the sixth resistance element is 1 ≤ R7/R6 ≤ 10, and that a ratio C4/C3 of an electrostatic capacitance C4 of the fourth capacitor to an electrostatic capacitance C3 of the third capacitor is 5 ≤ C4/C3 ≤ 35.

The analog system may include an analog output terminal through which the analog-format biological sound signal is output and to which an acoustic device that converts the biological sound signal into a sound wave is connected, and the digital system may include an analog-to-digital converter that converts the biological sound signal into the digital signal.

The analog system may include an adjustment circuit that adjusts an amplitude of the biological sound signal, and the digital system may include a communication circuit that transmits the digital signal to the outside via wired or wireless communication.

According to the disclosed technology, there is provided an electronic stethoscope capable of improving the sound quality of the detected biological sounds and auscultation sounds.

### BRIEF DESCRIPTION OF THE DRAWINGS

Fig. 1 is a circuit block diagram showing an example of a configuration of an electronic stethoscope according to an embodiment of the disclosed technology.
Fig. 2 is a cross-sectional view showing an example of a configuration of a biological sound sensor according to the embodiment of the disclosed technology.
Fig. 3 is a circuit diagram showing an example of a configuration of a first preamplifier according to the embodiment of the disclosed technology.
Fig. 4 is a circuit diagram showing an example of a configuration of a second preamplifier according to the embodiment of the disclosed technology.
Fig. 5 is a diagram schematically showing a frequency characteristic of a filter unit according to the embodiment of the disclosed technology.
Fig. 6 is a circuit diagram showing an example of a configuration of a second preamplifier according to another embodiment of the disclosed technology.

### DESCRIPTION OF THE PREFERRED EMBODIMENTS

Hereinafter, an example of embodiments of the present invention will be described with reference to the drawings. Note that in each drawing, the same or equivalent constituent elements and parts are designated by the same reference numerals, and overlapping description will not be repeated as appropriate.

### [First Embodiment]

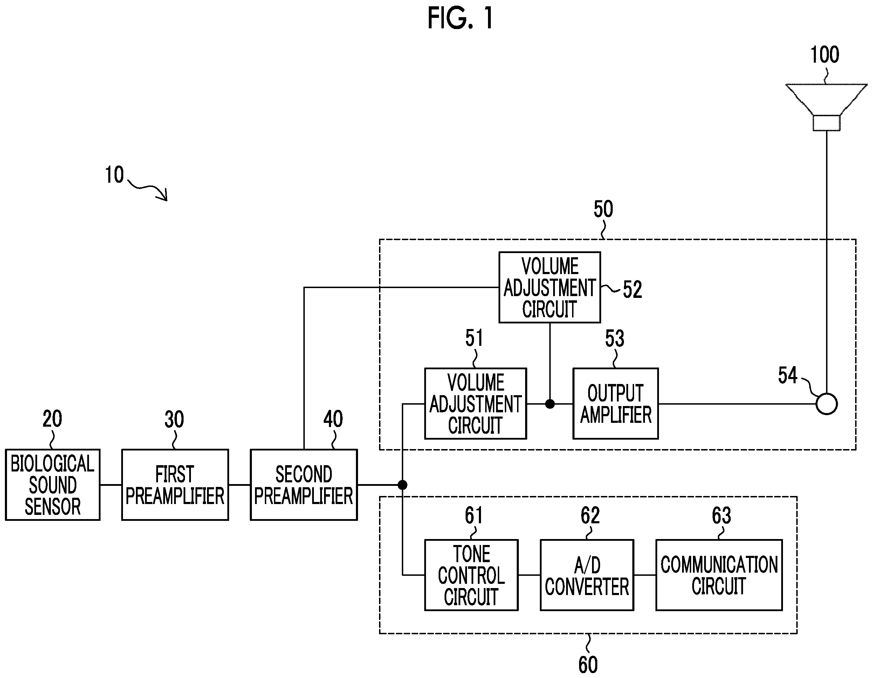

Fig. 1 is a circuit block diagram showing an example of the configuration of an electronic stethoscope 10 according to the embodiment of the disclosed technology. An electronic stethoscope 10 comprises a biological sound sensor 20 that detects a biological sound and that outputs an analog-format biological sound signal, an analog system 50 that processes the biological sound signal without converting the biological sound signal into a digital signal and that outputs the biological sound signal to an outside, and a digital system 60 that converts the biological sound signal into the digital signal and that outputs the biological sound signal to the outside. In addition, the electronic stethoscope 10 comprises a first preamplifier 30 that amplifies the biological sound signal and a second preamplifier 40 that attenuates a high-frequency component included in the biological sound signal, and an output signal of the second preamplifier 40 is distributed to both the analog system 50 and the digital system 60.

The analog system 50 includes a volume adjustment circuit 51, an automatic level control circuit 52, an output amplifier 53, and an analog output terminal 54. An acoustic device 100 that converts the biological sound signal into a sound wave, such as a headphone and an earphone, is connected to the analog output terminal 54. The digital system 60 includes a tone control circuit 61, an analog-to-digital converter 62, and a communication circuit 63. Hereinafter, each of the above-described constituent elements of the electronic stethoscope 10 will be described in detail.

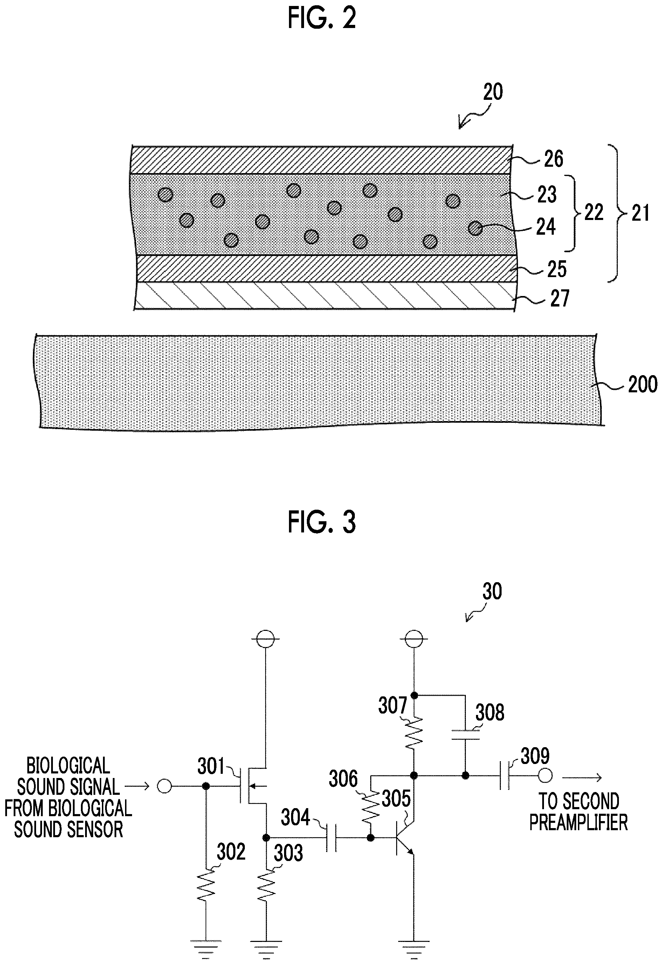

The biological sound sensor 20 detects the biological sound such as a heart sound and a respiratory sound of a subject to be examined and outputs the biological sound signal which is an analog-format electrical signal. Fig. 2 is a cross-sectional view showing an example of the configuration of the biological sound sensor 20. The biological sound sensor 20 includes a piezoelectric film 21 that converts vibration into an electrical signal and a protective layer 27. In a case where the biological sound is detected by the biological sound sensor 20, a laminate consisting of the piezoelectric film 21 and the protective layer 27 is in contact with a skin of a subject to be examined 200. The laminate consisting of the piezoelectric film 21 and the protective layer 27 has flexibility and can be brought into close contact with the skin of the subject to be examined 200. As a result, the detection sensitivity of the biological sound can be increased.

The piezoelectric film 21 includes a piezoelectric layer 22, a first electrode 25, and a second electrode 26. The piezoelectric layer 22 is sandwiched between the first electrode 25 and the second electrode 26. The piezoelectric layer 22 expands and contracts in an in-plane direction in response to the biological sound emitted from the subject to be examined 200. As a result, a voltage is generated between the first electrode 25 and the second electrode 26. In the present embodiment, the piezoelectric layer 22 is composed of a polymer-based piezoelectric composite material obtained by dispersing piezoelectric particles 24 in a viscoelastic matrix 23 consisting of a polymer material having viscoelasticity at room temperature. The piezoelectric particles 24 may be uniformly dispersed with regularity or may be irregularly dispersed, in the viscoelastic matrix 23.

As the viscoelastic matrix 23, it is possible to suitably use, for example, polymer materials, such as cyanoethylated polyvinyl alcohol (cyanoethylated PVA), polyvinyl acetate, polyvinylidene chloride-co-acrylonitrile, polystyrene-vinyl polyisoprene block copolymer, polyvinyl methyl ketone, and polybutyl methacrylate.

The piezoelectric particles 24 may be, for example, ceramic particles having a perovskite-type crystal structure. As the piezoelectric particles 24, it is possible to suitably use, for example, lead zirconate titanate, lead lanthanum zirconate titanate, barium titanate, a solid solution of barium titanate and bismuth ferrite, or the like.

The thickness of each of the first electrode 25 and the second electrode 26 is not particularly limited, but is preferably thin in order to ensure the flexibility of the piezoelectric film 21 and is preferably 1 µm or less, for example. The thicknesses of the first electrode 25 and the second electrode 26 may be the same or different. As materials for the first electrode 25 and the second electrode 26, it is possible to suitably use, for example, a thin film of copper (Cu) or aluminum (Al) deposited by vacuum deposition, a conductive polymer, or the like.

The protective layer 27 has a function of protecting the piezoelectric film 21. In addition, the protective layer 27 also functions as a buffer layer that alleviates a difference in acoustic impedance between the piezoelectric film 21 and the subject to be examined 200. That is, the protective layer 27 has an intermediate acoustic impedance between the acoustic impedance of the piezoelectric film 21 and the acoustic impedance of the subject to be examined 200. This makes it possible to detect the biological sound in a state of high sound quality. As the protective layer 27, it is possible to suitably use, for example, an elastomer material, a silicone resin, a silicone rubber, a urethane rubber, a natural rubber, a styrene-butadiene rubber, a chloroprene rubber, an acrylonitrile rubber, a butyl rubber, an ethylene propylene rubber, a fluoro-rubber, a chlorosulfonated polyethylene rubber, or the like.

As described above, the biological sound sensor 20 according to the present embodiment contains the polymer-based piezoelectric composite material obtained by dispersing the piezoelectric particles in the viscoelastic matrix consisting of the polymer material having viscoelasticity at room temperature and has flexibility, so that it can be brought into close contact with the skin of the subject to be examined 200. As a result, the detection sensitivity in the frequency range (for example, 1 KHz or less) of the biological sound is increased as compared with a biological sound sensor containing piezoelectric ceramics having no flexibility. In addition, with the biological sound sensor 20 according to the present embodiment, the dielectric loss can be increased by about 5 times to 10 times as compared with the biological sound sensor containing piezoelectric ceramics. The increase in dielectric loss promotes the conversion of vibration into heat, thereby suppressing reverberant sound. With the biological sound sensor 20 according to the present embodiment, the biological sound can be detected with high sound quality as compared with the biological sound sensor containing piezoelectric ceramics, so that digital signal processing is not required in order to improve the sound quality. That is, the biological sound with high sound quality can be obtained even in a case where the biological sound is reproduced by performing only analog signal processing on the biological sound signal output from the biological sound sensor 20. The biological sound signal output from the biological sound sensor 20 is supplied to the first preamplifier.

Fig. 3 is a circuit diagram showing an example of the configuration of the first preamplifier 30. The first preamplifier 30 has a function of amplifying the biological sound signal output from the biological sound sensor 20. The first preamplifier 30 includes transistors 301 and 305, resistance elements 302, 303, 306, and 307, and capacitors 304, 308, and 309.

The transistor 301 is a metal-oxide-semiconductor field effect transistor (MOSFET), and a source thereof is connected to a power supply potential, a drain thereof is connected to one end of the resistance element 303, and a gate thereof is connected to one end of the resistance element 302. The other end of the resistance element 303 and the drain of the transistor 301 are each connected to a ground potential. The transistor 301 and the resistance elements 302 and 303 constitute a so-called source follower circuit.

The gate of the transistor 301 is an input terminal into which the biological sound signal from the biological sound sensor 20 is input, that is, an input terminal of the first preamplifier 30. It is preferable that an input impedance in the input terminal is high. The reason for the above is as follows: in a case where the input impedance becomes excessively low, the charge may be dissipated and the amplitude of the biological sound signal becomes small, and in a case where signal amplification is performed to compensate for this, the noise component may also be amplified, resulting in a decrease in SN ratio. An input impedance Z of the first preamplifier 30 is preferably 50 kS2 ≤ Z ≤ 10 MS2. By setting the input impedance Z of the first preamplifier 30 to 50 kQ or more, a decrease in the SN ratio can be suppressed. By setting the input impedance Z of the first preamplifier 30 to 10 MΩ or less, noise immunity can be ensured. The input impedance of the first preamplifier 30 is determined by the input impedance of the transistor 301 and a resistance value of the resistance element 302. Therefore, the transistor 301 is preferably a MOSFET having a high input impedance.

The biological sound signal, which is impedance-converted by the source follower circuit including the transistor 301 and the resistance elements 302 and 303, is amplified by the amplification circuit including the transistor 305, the resistance elements 306 and 307, and the capacitors 304 and 308. The transistor 305 is a bipolar NPN transistor, and a collector thereof is connected to the power supply potential via a CR parallel circuit in which the resistance element 307 and the capacitor 308 are connected in parallel, a base thereof is connected to the drain of the transistor 301 via the capacitor 304, and an emitter thereof is connected to the ground potential. One end of the resistance element 306 is connected to the collector of the transistor 305, and the other end thereof is connected to the base of the transistor 305. One end of the capacitor 309 is connected to the collector of the transistor 305, and the other end thereof is an output end of the first preamplifier 30. The capacitors 304 and 309 each function as a coupling capacitor that cuts off a direct-current component. By configuring the amplification circuit in the first preamplifier 30 with a so-called emitter-grounded amplification circuit as described above, it is possible to obtain characteristics of high gain and low noise. The amplified biological sound signal output from the output end of the first preamplifier 30 is supplied to the second preamplifier 40.

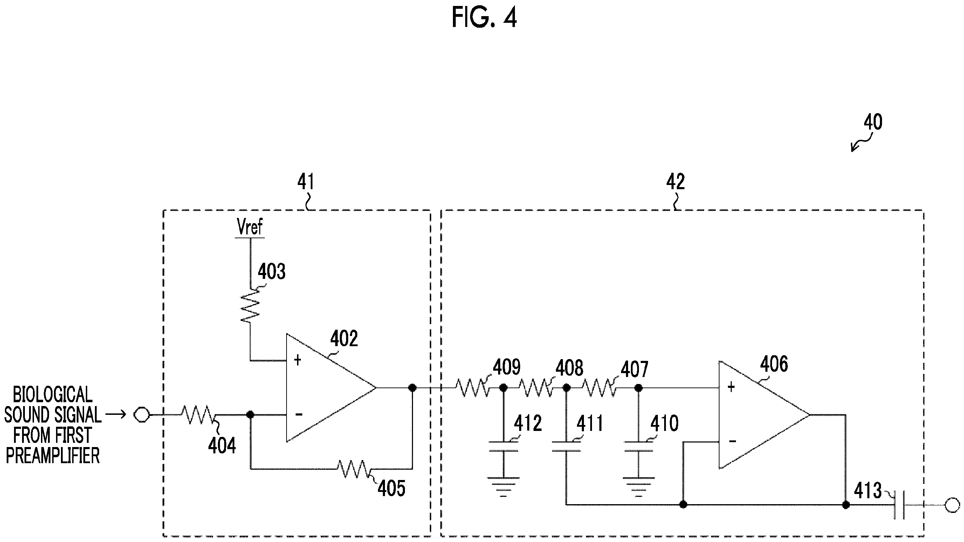

Fig. 4 is a circuit diagram showing an example of the configuration of the second preamplifier 40. The second preamplifier 40 has a function of further amplifying the biological sound signal supplied from the first preamplifier 30 and a function of attenuating a high-frequency component included in the biological sound signal. The second preamplifier 40 includes an amplification unit 41 that has a signal amplification function and a filter unit 42 that has a function of attenuating the high-frequency component.

The amplification unit 41 includes an operational amplifier 402 and resistance elements 403, 404, and 405. One end of the resistance element 403 is connected to a reference voltage Vref, and the other end thereof is connected to a non-inverting input terminal of the operational amplifier 402. One end of the resistance element 404 is connected to the output end of the first preamplifier 30, and the other end thereof is connected to an inverting input terminal of the operational amplifier 402. One end of the resistance element 405 is connected to the inverting input terminal of the operational amplifier 402, and the other end thereof is connected to an output terminal of the operational amplifier 402. The operational amplifier 402 and the resistance elements 403, 404, and 405 constitute a so-called inverting amplification circuit. The operational amplifier 402 is an example of a first operational amplifier in the disclosed technology. The resistance element 404 is an example of a first resistance element in the disclosed technology. The resistance element 405 is an example of a second resistance element in the disclosed technology. An amplification factor in the amplification unit 41 is appropriately set according to an amplification factor in the first preamplifier 30. The amplification factor in the amplification unit 41 corresponds to a ratio R2/R1 of a resistance value R2 of the resistance element 405 to a resistance value R1 of the resistance element 404. The ratio R2/R1 is preferably 1 ≤ R2/R1 ≤ 10. The biological sound signal amplified by the amplification unit 41 is supplied to the filter unit 42.

The filter unit 42 includes an operational amplifier 406, resistance elements 407, 408, and 409, and capacitors 410, 411, 412, and 413. One end of the resistance element 407 is connected to a non-inverting input terminal of the operational amplifier 406. One end of the resistance element 408 is connected to the other end of the resistance element 407. One end of the resistance element 409 is connected to the other end of the resistance element 408, and the other end thereof is connected to the output terminal of the operational amplifier 402. One end of the capacitor 410 is connected to the non-inverting input terminal of the operational amplifier 406, and the other end thereof is connected to the ground potential. One end of the capacitor 411 is connected to a connection portion between the resistance element 407 and the resistance element 408, and the other end thereof is connected to an inverting input terminal and an output terminal of the operational amplifier 406. One end of the capacitor 412 is connected to a connection portion between the resistance element 408 and the resistance element 409. One end of the capacitor 413 is connected to the output terminal of the operational amplifier 406, and the other end thereof is an output end of the second preamplifier 40.

The operational amplifier 406, the resistance elements 407 and 408, and the capacitors 410 and 411 constitute an active low-pass filter of a second-order voltage-controlled voltage source type (VCVS type). The resistance element 409 and the capacitor 412 constitute a first-order passive low-pass filter. The operational amplifier 406 is an example of a second operational amplifier in the disclosed technology. The resistance element 407 is an example of a third resistance element in the disclosed technology. The resistance element 408 is an example of a fourth resistance element in the disclosed technology. The capacitor 410 is an example of a first capacitor in the disclosed technology. The capacitor 411 is an example of a second capacitor in the disclosed technology.

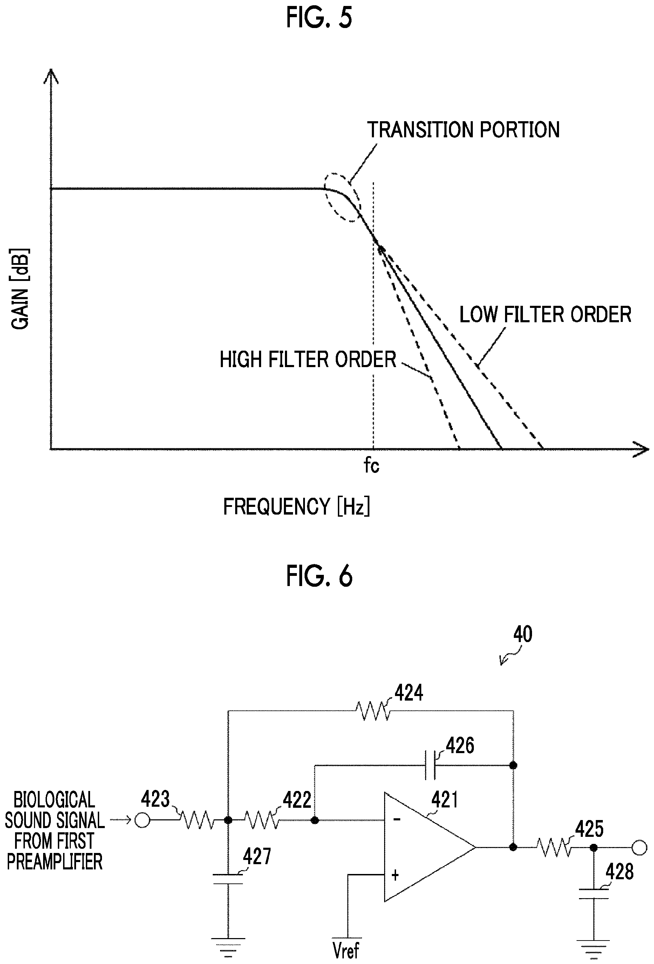

Fig. 5 is a diagram schematically showing a frequency characteristic of the filter unit 42. In Fig. 5, the horizontal axis represents a frequency of a signal input to the filter unit 42, and the vertical axis represents a gain in the filter unit 42. As shown in Fig. 5, the filter unit 42 attenuates a high-frequency component of the input signal. A cutoff frequency fc in the filter unit 42 is preferably 1 kHz ≤ fc ≤ 3 kHz. As a result, it is possible to remove components other than the biological sound included in the biological sound signal.

Ideally, as the frequency characteristic of the filter unit 42, the gain changes abruptly before and after the cutoff frequency f_{C}. That is, it is preferable that the angle of a transition portion of the frequency characteristic is 90 degrees and that an attenuation slope A is steep. The attenuation slope A is defined as a change amount ΔG of the gain with respect to a change amount Δf of the frequency, where A = |ΔG| / |Δf|.

The attenuation slope A of the filter unit 42 is preferably 12 dB/oct ≤ A ≤ 36 dB/oct. The attenuation slope A of the filter unit 42 can be controlled by the order of the low-pass filter. In the present embodiment, the filter unit 42 is configured as a multi-stage third-order low-pass filter, which is composed of a first-order low-pass filter composed of the resistance element 409 and the capacitor 412, and a second-order low-pass filter composed of the operational amplifier 406, the resistance elements 407 and 408, and the capacitors 410 and 411. According to this configuration, the attenuation slope A is 18 dB/oct.

The filter unit 42 may be composed of only the second-order low-pass filter. According to this configuration, the attenuation slope A is 12 dB/oct. Alternatively, the filter unit 42 may be configured as a multi-stage fifth-order low-pass filter, which is composed of one stage of the first-order low-pass filter and two stages of the second-order low-pass filter. According to this configuration, the attenuation slope A is 30 dB/oct. Alternatively, the filter unit 42 may be configured as a multi-stage sixth-order low-pass filter, which is composed of two stages of the first-order low-pass filter and two stages of the second-order low-pass filter. According to this configuration, the attenuation slope A is 36 dB/oct.

The angle of the transition portion of the frequency characteristic of the filter unit 42 can be brought closer to 90 degrees by increasing a ratio C2/C1 of an electrostatic capacitance C2 of the capacitor 411 to an electrostatic capacitance C1 of the capacitor 410. The ratio C2/C1 is preferably 3 ≤ C2/C1 ≤ 15 and more preferably 3 ≤ C2/C1 ≤ 10.

As described above, the biological sound signal output from the biological sound sensor 20 is subjected to preprocessing including amplification processing and filtering processing in the first preamplifier 30 and the second preamplifier 40. The biological sound signal that has been subjected to the preprocessing, that is, the output signal of the second preamplifier 40 is distributed to both the analog system 50 and the digital system 60.

The volume adjustment circuit 51 has a function of adjusting the amplitude of the biological sound signal based on a user's operation through an input unit for volume adjustment (not shown) provided in the electronic stethoscope 10. That is, the volume adjustment circuit 51 adjusts the volume of the biological sound to be emitted from the acoustic device 100. The volume adjustment circuit 51 is composed of an analog circuit that processes the biological sound signal as an analog signal. The volume adjustment circuit 51 is an example of an adjustment circuit in the disclosed technology.

The automatic level control circuit 52 has a function of suppressing the amplitude of the biological sound signal in a case where the amplitude of the biological sound signal is equal to or higher than a certain level. As a result, it is possible to prevent the sudden excessive sound from being reproduced. The automatic level control circuit 52 may be composed of an analog circuit that processes the biological sound signal as an analog signal, and may include, for example, a transistor in which an output signal of the amplification unit 41 of the second preamplifier 40 is input to a gate.

The output amplifier 53 amplifies the biological sound signal to a power suitable for output as audio. The output amplifier 53 is composed of an analog circuit that processes the biological sound signal as an analog signal. An output signal of the output amplifier 53 is supplied to the acoustic device 100 connected to the analog output terminal 54. The acoustic device 100 is a device that converts the biological sound signal into a sound wave, such as a headphone or an earphone. The biological sound detected by the electronic stethoscope 10 is perceived by the user via the acoustic device 100.

The tone control circuit 61 has a function of controlling the balance between the high-frequency component and a low-frequency component of the biological sound signal based on an operation from the user through an input unit for tone adjustment (not shown) provided in the electronic stethoscope 10.

The analog-to-digital converter 62 converts the analog-format biological sound signal into a digital signal and outputs the digital signal.

The communication circuit 63 transmits the digital-format biological sound signal to an external device through at least one method of wired communication or wireless communication. The wireless communication method may include, for example, Bluetooth (registered trademark). In addition, the communication circuit 63 may be provided with a communication port for transmitting the digital-format biological sound signal through wired communication. The communication port may be, for example, a universal serial bus (USB) port. The digital-format biological sound signal transmitted from the communication circuit 63 can be reproduced as audio or an image (signal waveform) in the external device.

As described above, the electronic stethoscope 10 according to the embodiment of the disclosed technology includes the analog system 50 that processes the biological sound signal output from the biological sound sensor 20 without converting the biological sound signal into the digital signal and that outputs the biological sound signal to the outside, and the digital system 60 that converts and outputs the biological sound signal into the digital signal. The biological sound sensor 20 contains a polymer-based piezoelectric composite material obtained by dispersing the piezoelectric particles in the viscoelastic matrix consisting of the polymer material having viscoelasticity at room temperature.

With the biological sound sensor 20 according to the present embodiment, the biological sound can be detected with high sound quality as compared with the biological sound sensor containing piezoelectric ceramics, so that digital signal processing is not required in order to improve the sound quality. In the electronic stethoscope 10 according to the present embodiment, in the analog system 50, the biological sound signal is processed as an analog signal without being converted into the digital signal and is supplied to the acoustic device 100. Therefore, the degradation of the biological sound signal due to the sampling and the quantization is avoided, and the acoustic device 100 can reproduce the natural biological sound without discomfort. That is, with the electronic stethoscope 10 according to the present embodiment, it is possible to improve the sound quality of the detected biological sounds and auscultation sounds. In addition, the electronic stethoscope 10 comprises not only the analog system 50 but also the digital system 60, which facilitates various types of processing, such as storage, transmission, visualization, processing, and analysis of the biological sound signal.

In addition, in the electronic stethoscope 10 according to the present embodiment, the preprocessing including the amplification processing and the filtering processing performed on the biological sound signal output from the biological sound sensor 20 is performed by the first preamplifier 30 and the second preamplifier 40. In the preamplifier that performs the preprocessing on the biological sound signal, it is required to receive the biological sound signal at a high input impedance in order to increase the SN ratio, but it is not easy to achieve desired signal processing while ensuring the high input impedance using a single amplification circuit. In the electronic stethoscope 10 according to the present embodiment, the first preamplifier 30 has a function of receiving the biological sound signal at a high input impedance, the second preamplifier 40 has a filtering function, and both the first preamplifier 30 and the second preamplifier 40 have a signal amplification function. This makes it possible to perform desired signal processing while ensuring a high input impedance.

### [Second Embodiment]

Fig. 6 is a circuit diagram showing an example of the configuration of the second preamplifier 40 according to the second embodiment of the disclosed technology. The second preamplifier 40 according to the present embodiment includes an operational amplifier 421, resistance elements 422, 423, 424, and 425, and capacitors 426, 427, and 428.

One end of the resistance element 422 is connected to an inverting input terminal of the operational amplifier 421. One end of the resistance element 423 is connected to the other end of the resistance element 422, and the other end thereof is connected to the output end of the first preamplifier 30. One end of the resistance element 424 is connected to an output terminal of the operational amplifier 421, and the other end thereof is connected to a connection portion between the resistance element 422 and the resistance element 423. One end of the resistance element 425 is connected to the output terminal of the operational amplifier 421, and the other end thereof is the output end of the second preamplifier 40. One end of the capacitor 426 is connected to the inverting input terminal of the operational amplifier 421, and the other end thereof is connected to the output terminal of the operational amplifier 421.

One end of the capacitor 427 is connected to the connection portion between the resistance element 422 and the resistance element 423, and the other end thereof is connected to the ground potential. One end of the capacitor 428 is connected to the other end of the resistance element 425, and the other end thereof is connected to the ground potential. A non-inverting input terminal of the operational amplifier 421 is connected to the reference voltage Vref.

The operational amplifier 421, the resistance elements 422, 423, and 424, and the capacitors 426 and 427 constitute a second-order multiple feedback active low-pass filter. The resistance element 425 and the capacitor 428 constitute the first-order passive low-pass filter. The operational amplifier 421 is an example of a third operational amplifier in the disclosed technology. The resistance element 422 is an example of a fifth resistance element in the disclosed technology. The resistance element 423 is an example of a sixth resistance element in the disclosed technology. The resistance element 424 is an example of a seventh resistance element in the disclosed technology. The capacitor 426 is an example of a third capacitor in the disclosed technology. The capacitor 427 is an example of a fourth capacitor in the disclosed technology.

An amplification factor in the second preamplifier 40 according to the present embodiment corresponds to a ratio R7/R6 of a resistance value R7 of the resistance element 424 to a resistance value R6 of the resistance element 423. The ratio R7/R6 is preferably 1 ≤ R7/R6 ≤ 10. The angle of the transition portion of the frequency characteristic of the second preamplifier 40 according to the present embodiment can be brought closer to 90 degrees by increasing a ratio C4/C3 of an electrostatic capacitance C4 of the capacitor 427 to an electrostatic capacitance C3 of the capacitor 426. The ratio C4/C3 is preferably 5 ≤ C4/C3 ≤ 35 and more preferably 20 ≤ C4/C3 ≤ 30.

With the second preamplifier 40 according to the present embodiment, the same functions as those of the second preamplifier 40 (see Fig. 4) according to the first embodiment can be exhibited.

The disclosure of JP2021-030783 filed on February 26, 2021 is incorporated in the present specification by reference in its entirety. In addition, all documents, patent applications, and technical standards described in the present specification are incorporated in the present specification by reference to the same extent as in a case where the individual documents, patent applications, and technical standards have been specifically and individually stated to be incorporated by reference.

## Claims

1. An electronic stethoscope comprising:
a biological sound sensor that detects a biological sound and outputs an analog-format biological sound signal;
an analog system that processes the biological sound signal without converting the biological sound signal into a digital signal and outputs the biological sound signal to an outside; and
a digital system that converts the biological sound signal into the digital signal and outputs the biological sound signal.

2. The electronic stethoscope according to claim 1,
wherein the biological sound sensor contains a polymer-based piezoelectric composite material obtained by dispersing piezoelectric particles in a viscoelastic matrix consisting of a polymer material having viscoelasticity at room temperature.

3. The electronic stethoscope according to claim 1 or 2, further comprising:
a first preamplifier that amplifies the biological sound signal; and
a second preamplifier that attenuates a high-frequency component included in the biological sound signal,
wherein an output signal of the second preamplifier is distributed to the analog system and the digital system.

4. The electronic stethoscope according to claim 3,
wherein the first preamplifier has an input impedance Z of 50 kS2 ≤ Z ≤ 10 MΩ, and
the second preamplifier has a cutoff frequency fc of 1 kHz ≤ fc ≤ 3 kHz and an attenuation slope A of 12 dB/oct ≤ A ≤ 36 dB/oct.

5. The electronic stethoscope according to claim 3 or 4,
wherein the second preamplifier includes an amplification unit and a filter unit,
the amplification unit includes
a first operational amplifier having a first inverting input terminal, a first non-inverting input terminal, and a first output terminal,
a first resistance element of which one end is connected to an output end of the first preamplifier and the other end is connected to the first inverting input terminal, and
a second resistance element of which one end is connected to the first inverting input terminal and the other end is connected to the first output terminal, and
the filter unit includes
a second operational amplifier having a second inverting input terminal, a second non-inverting input terminal, and a second output terminal,
a third resistance element of which one end is connected to the second non-inverting input terminal,
a first capacitor of which one end is connected to the non-inverting input terminal and the other end is connected to a ground potential,
a fourth resistance element of which one end is connected to the other end of the third resistance element, and
a second capacitor of which one end is connected to a connection portion between the third resistance element and the fourth resistance element and the other end is connected to the second inverting input terminal and the second output terminal.

6. The electronic stethoscope according to claim 5,
wherein a ratio R2/R1 of a resistance value R2 of the second resistance element to a resistance value R1 of the first resistance element is 1 ≤ R2/R1 ≤ 10, and
a ratio C2/C1 of an electrostatic capacitance C2 of the second capacitor to an electrostatic capacitance C 1 of the first capacitor is 3 ≤ C2/C1 ≤ 15.

7. The electronic stethoscope according to claim 3 or 4,
wherein the second preamplifier includes
a third operational amplifier having a third inverting input terminal, a third non-inverting input terminal, and a third output terminal,
a fifth resistance element of which one end is connected to the third inverting input terminal,
a sixth resistance element of which one end is connected to the other end of the fifth resistance element,
a seventh resistance element of which one end is connected to the third output terminal and the other end is connected to a connection portion between the fifth resistance element and the sixth resistance element,
a third capacitor of which one end is connected to the third inverting input terminal and the other end is connected to the third output terminal, and
a fourth capacitor of which one end is connected to the connection portion between the fifth resistance element and the sixth resistance element and the other end is connected to a ground potential.

8. The electronic stethoscope according to claim 7,
wherein a ratio R7/R6 of a resistance value R7 of the seventh resistance element to a resistance value R6 of the sixth resistance element is 1 ≤ R7/R6 ≤ 10, and
a ratio C4/C3 of an electrostatic capacitance C4 of the fourth capacitor to an electrostatic capacitance C3 of the third capacitor is 5 ≤ C4/C3 ≤ 35.

9. The electronic stethoscope according to any one of claims 1 to 8,
wherein the analog system includes an analog output terminal through which the analog-format biological sound signal is output and to which an acoustic device that converts the biological sound signal into a sound wave is connected, and
the digital system includes an analog-to-digital converter that converts the biological sound signal into the digital signal.

10. The electronic stethoscope according to claim 9,
wherein the analog system includes an adjustment circuit that adjusts an amplitude of the biological sound signal, and
the digital system includes a communication circuit that transmits the digital signal to the outside via wired or wireless communication.
